# EUROPEAN PATENT SPECIFICATION

(11) **EP 2 328 519 B1**
(45) Date of publication and mention of the grant of the patent: **31.12.2014**
(21) Application number: 09758781.0
(22) Date of filing: 05.06.2009
(51) Int. Cl.: A61F 2/44

(54) **MODULAR ANTERIOR LOCKING INTERBODY CAGE**
MODULARER ANTERIORER ARRETIERENDER INTERKORPUS-KÄFIG
CAGE MODULAIRE INTERVERTÉBRALE DE VERROUILLAGE ANTÉRIEUR

(30) Priority: 05.06.2008 US 59181 P
(43) Date of publication of application: 08.06.2011
(73) Proprietor: Alphatec Spine, Inc., Carlsbad, CA 92008 (US)
(72) Inventor: AHN, Uri, Bedford NH (US)
(74) Representative: Mintz Levin Cohn Ferris Glovsky and Popeo LLP
(86) International application number: PCT/US2009/003421
(87) International publication number: WO 2009/148618

(56) References cited:
- EP-A- 1 470 803
- WO-A-2005/065596
- FR-A- 2 894 130
- US-A1- 2008 051 890

## Description

### BACKGROUND OF THE INVENTION

### Field of the Invention

The present invention is directed to systems, methods, and devices applicable to spinal surgery. More specifically, the present invention is directed to a modular spinal spacer designed to accommodate the vascular anatomy for use by medical personnel (i.e., doctor) in spinal and other surgical procedures. In some embodiments of the present invention relates to a modular spinal spacer for insertion into a disk space defined between two adjacent vertebrae near vascular anatomy, in order to restore an appropriate height between the vertebrae and to allow bone fusion to take place between adjacent vertebrae.

### Background of the Invention

Vertebrae are the individual irregular bones that make up the spinal column (aka ischis)--a flexuous and flexible column. There are normally thirty-three vertebrae in humans, including the five that are fused to form the sacrum (the others are separated by intervertebral discs) and the four coccygeal bones which form the tailbone. The upper three regions comprise the remaining 24, and are grouped under the names cervical (7 vertebrae), thoracic (12 vertebrae) and lumbar (5 vertebrae), according to the regions they occupy. This number is sometimes increased by an additional vertebra in one region, or it may be diminished in one region, the deficiency often being supplied by an additional vertebra in another. The number of cervical vertebrae is, however, very rarely increased or diminished.

A typical vertebra consists of two essential parts: an anterior (front) segment, which is the vertebral body; and a posterior part-the vertebral (neural) arch- which encloses the vertebral foramen. The vertebral arch is formed by a pair of pedicles and a pair of laminae, and supports seven processes, four articular, two transverse, and one spinous, the latter also being known as the neural spine.

When the vertebrae are articulated with each other, the bodies form a strong pillar for the support of the head and trunk, and the vertebral for amina constitute a canal for the protection of the medulla spinalis (spinal cord), while between every pair of vertebrae are two apertures, the intervertebral foramina, one on either side, for the transmission of the spinal nerves and vessels.

Conventional interbody spacer assemblies are used in spinal fusion procedures to repair damaged or incorrectly articulating vertebrae. Conventional interbody spacer assemblies come in different cross sections. Some spacer assemblies may be hollow and may include openings in the side(s) thereof to provide access for bone matter growth. The use on interbody spacers are primarily to support the anterior load of the spinal column and provide a method for insertion and containment of bone graft material to facilitate spinal fusion. Often these spacers are used in conjunction with supplemental fixation in the form of pedicle screws or anterior plate systems. Examples of interbody spacer assemblies can be seen in EP1470803 which describes an intervertebral implant and us2008/051890 which describes vertebral implants for spacing apart vertebral members.

Historically one of the failure modes of interbody spacers used in combination with anterior plate systems particularly in the lumbar spine in one of placing the anterior plate due to the vascular system lying directly over the area of interest. This has been previously addressed by surgically mobilizing the vascular structures or particularly in the upper lumbar levels avoiding the use of anterior lumbar plate's altogether and utilizing posterior supplemental instrumentation. Some implant designs have integrated features that provide for integrated supplemental fixation such as spikes, protrusions, screws, once installed but generally do not provide the same revel of rigidity as a plate creating a paradoxical relationship where implant manufacturers must choose between either making the implant system easier to insert or making the implant system more effective in stabilize the spine top facilitate fusion.

There exists a need for further improvements in the field of spinal spacer assemblies of the present type that are designed to avoid the vascular structures.

### BRIEF SUMMARY OF THE INVENTION

In a first aspect, embodiments of the present invention provide a modular spinal implant system for use between adjacent vertebrae near vascular anatomy. The system includes an implant configured to fit between adjacent vertebrae, the implant having annular side walls with upper and lower surfaces configured to enclose a hollow interior, and an attachment plate rotatably coupled to the implant and configured to rotate to variable orientations relative to the implant to avoid the vascular anatomy, the attachment plate having a superior portion that is narrower than an inferior portion, the attachment plate having at least one vertebra attachment hole configured for attaching to at least one adjacent vertebrae using one or more bone screws.

In many embodiments, the attachment plate is selected from a variety of attachment plates configured to avoid the vascular anatomy proximate the vertebrae.

In many embodiments, the inferior portion includes one or more vertebra attachment holes.

In many embodiments, the superior portion includes one vertebra attachment hole and the inferior portion includes two vertebra attachment holes.

In many embodiments, the implant material is selected from the group consisting of titanium, stainless steel, cobalt-chromium, carbon, PEEK (polyethylketone), graphite, woven carbon, Kevlar, and other suitable synthetic material.

In many embodiments, the implant is made of a non metal synthetic material. According to the invention, the implant further includes one or more metal plates integrally formed within an anterior portion of the annular side wall

In many embodiments, the attachment plate is made from titanium, stainless steel, or cobalt-chromium.

In many embodiments, mating surfaces between the attachment plate and implant include an interlock configuration.

In many embodiments, the system further includes a bone autograft, allograft or a bone graft substitute positioned within the hollow interior of the implant.

### BRIEF DESCRIPTION OF THE DRAWINGS

FIG. 1 shows a top view of one embodiment of an oval shaped body or cage.
FIG. 2 shows a top view of a "D" shaped body.
FIG. 3A shows a L5/S1 anterior view of a modular anterior locking interbody cage (MALIC) system in place between vertebrae proximate vascular structures.
FIG. 3B shows one embodiment of attaching a plate to the implant.
FIG. 4A shows one example of an anterior view of the spine in which a vascular portion may require retraction for implantation and attachment of an attachment plate.
FIG. 4B shows retraction of the vascular portion.
FIG. 4C-4E show different sizes and shapes of attachment plates to attach to an implant that may be used to avoid the vascular.
FIG. 5 shows examples of surface treatment of the plate and implant where they join.
FIG. 6A shows a top view of one embodiment an implant that is a non metal synthetic material (NMSM)/metal amalgam.
FIGs. 6B-6E show other embodiments of a NMSM/metal amalgam implant.
FIG. 7 shows a view of a lateral x-ray showing an implant of FIG. 6A positioned within between adjacent vertebrae.

### DETAILED DESCRIPTION OF THE INVENTION

One or more detailed embodiments of the present invention are disclosed herein; however, it is to be understood that the disclosed embodiments are merely exemplary of the invention, which may be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure.

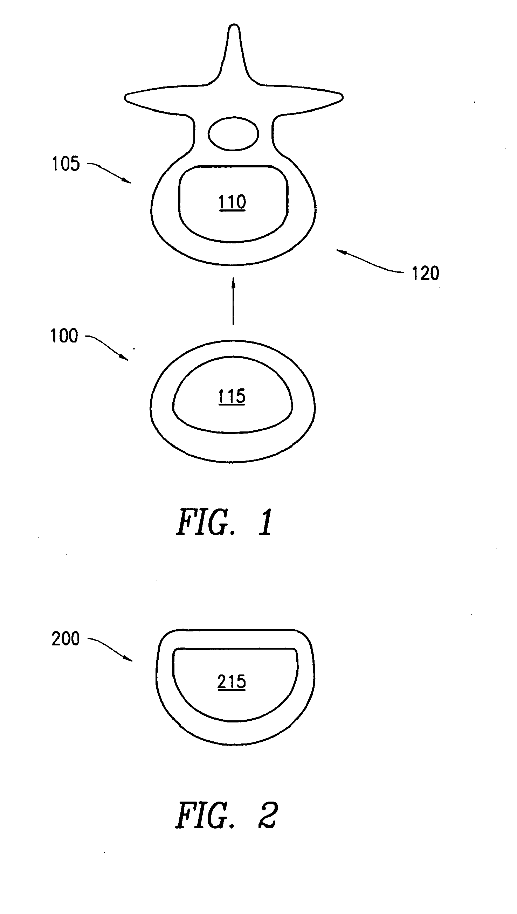

FIG. 1 shows one embodiment of an anterior locking interbody cage for use as an anterior interbody fusion device 100 in the lumbar spine 105. The disc interspace is shaped like a "D" 110. The device 100 has a generally oval or circular shaped body or cage, when viewed from above, having an annular wall enclosing a hollow interior or area 115 that would permit bony growth into spinal bones above and below the device when implanted. The hollow area 115 would be filled with bone autograft, allograft or a bone graft substitute.

FIG. 2 shows a "D" shaped body 200 body or cage, when viewed from above, having an annular wall enclosing a hollow interior or area 215 that would permit bony growth into spinal bones above and below the device when implanted. The hollow area 215 would be filled with bone autograft, allograft or a bone graft substitute.

There are some advantages using the oval or circular shape shown in FIG. 1 over "D" shape 200 shown in FIG. 2. For example, the "D" shaped device can only be placed in the disc space in one orientation. The "D" shape has posterior corners that can impinge on the aortic/venous iliac vessels. The "D" design is not suitable for a lateral approach 120. In contrast, an oval shaped device permits more implant options, so that the device could be placed in different rotational orientations within the spine, and would have the advantage of sliding safely past aortic/venous iliac vessels. The oval shaped device 100 also allows variability to approach the disc space from multiple angles 120, again permitting variability to better accommodate the vascular anatomy, such as. For example, oval shaped device may also be placed into patients through a lateral approach 120, which a "D" design does not afford, the lateral approach being favored by many surgeons in an anterior approach to L4/5.

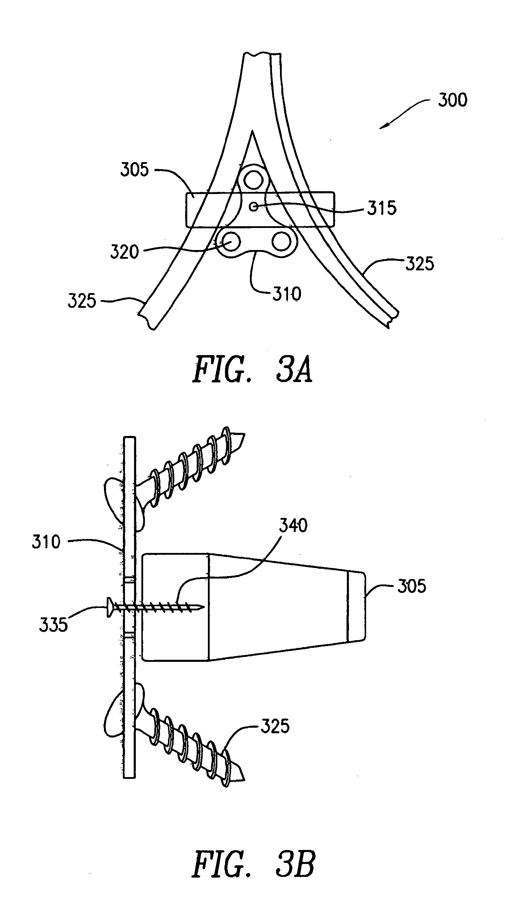

FIG. 3A shows a L5/S1 anterior view of one embodiment of a modular anterior locking interbody cage (MALIC) system 300 configured to be placed between vertebra. The system 300 includes an interbody fusion device or implant 305 and an attachment plate 310. The implant 305 may be made of a metal, a non metal synthetic material, or a NMSM/metal amalgam, discussed below. The attachment plates 310 are coupled to the implant with a screw, and would allow a variation of attachment plates 310 to be attached onto the implant 305, making the system 300 modular. The attachment plates are designed to accommodate the vascular 325 anatomy. Using a screw or other fixation means such as a rivet or snap locking mechanism for attachment with the implant allows the attachment plate to rotate to various orientations to avoid the vascular anatomy.. The attachment plate may be coupled to the implant either before, during, or after implantation. The screw attachment also allows the attachment plate to be removable from the body in case it needs to be replaced or repositioned.

The attachment plate 310 has a superior portion (anatomically cranial) and an inferior portion (anatomically caudal). The superior portion is narrower than the inferior portion. The attachment plate 310 in turn would have holes that would allow the placement of bone screws 320 into the vertebra above and/or below, locking the implant 305 in place. The attachment plate 310 may be made from metal, such as titanium, stainless steel or cobalt-chromium. The attachment plate 310 may also be made from high strength composites or plastics such as PEEK.

In addition, the attachment plate 310 adjacent to the device 305 may have a contouring that would allow a male/female counterpart contouring on a front surface of the device. This would allow a surface interlock that would resist rotational forces between the attachment plate 310 and the device 305. The primary reason for the attachment plate 310 shape is the complexity of the vascular anatomy, especially at the spinal levels superior to L5-S1, that can make access in one area of the spine easier than another. This would allow the surgeon a variety of attachment plates 310 to choose from, selecting the best shape to accommodate the complex vascular anatomy. By creating this modularity in attachable plates this device would have a variety of attachable plates that would accommodate different vascular anatomic challenges, allowing surgery to be performed in a safer manner. This would be done without sacrificing biomechanical strength and the plate would in turn lock onto the MALIC.

FIG. 3B is a side view showing one embodiment of attaching the attachment plate 310 to the implant 305. Screw 335 attaches the attachment plate 310 to the implant 305, in particular, attaching to the embedded metal plate 340 within the implant 305. Bone screws 325 are then used to attach the attachment plate to the vertebra above and/ or below implant 305.

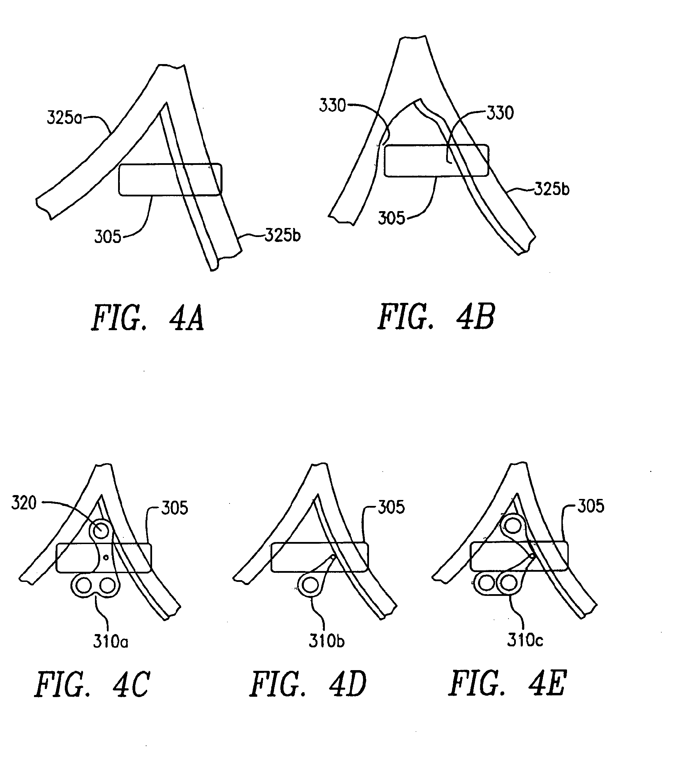

FIG. 4A shows one example of an anterior view of the spine in which a vascular portion requires retraction for implantation and attachment of an attachment plate 310 to a body 305 and adjacent vertebrae. In this example of the vertebral levels above L5-S1 area, the vascular portion 325 is draped over the left side of the vertebrae. The vascular 325a and/or 325b is retracted 330, such as shown in FIG. 4B, to make room for the attachment plate 310 to attach to the implant 305 and vertebrae. FIG. 4C-4E show different sizes and shapes of attachment plates 310 that may be used to avoid the vascular, having superior portions narrower than inferior portions. In FIG. 4C, attachment plate 310a may include provisions for one screw attaching to a vertebra above the implant 305 and two screws attaching to a vertebra below the implant 305. In FIG. 4D, attachment plate 310b attaches to a vertebra below the implant 305. In FIG. 4E, attachment plate 310c may include provisions for one screw attaching a vertebra above the implant 305 and two screws attaching to a vertebra below the implant 305.

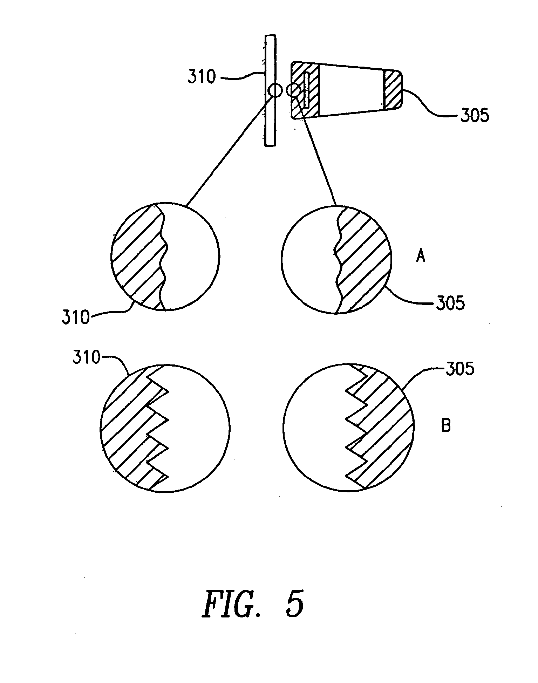

In some cases, it may be desirable to have surface treatment of the attachment plate 310 and implant 305 where they join. For example, FIG. 5 shows two examples A and B. The mating surfaces in A have adjacent irregular contouring and B have regular pyramidal male/female interlocking features to provide additional stability of the assemble components that may include rotational stability.

### non metal synthetic material/metal amalgam

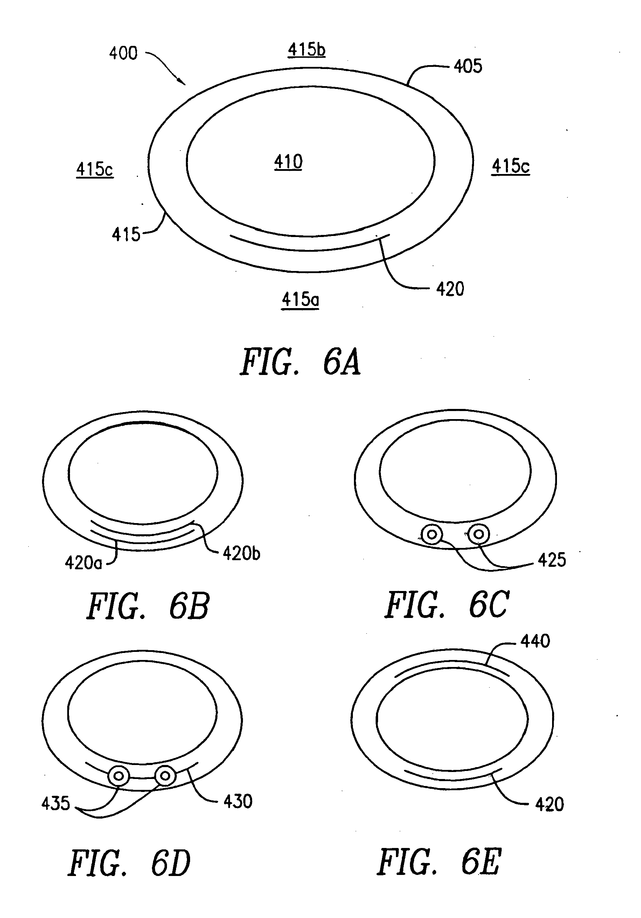

FIG. 6A shows one embodiment an implant 400, having a generally oval or circular shape similar to device 100, with an amalgam body of non metal synthetic material (NMSM) and metal material. In some embodiments, implant 400 may be used in place of implant 300 in the systems describe above. The device 400 includes an oval body 405 or cage with an annular wall 415 having upper and lower surfaces enclosing a central opening 410 or hollow interior. The upper and lower surfaces are configured to contact adjacent spine member and may have raised ridges projecting outwardly from each of the surfaces for engaging the spinal column. The annular wall 415 of the implant 405 includes an anterior portion 415a, a posterior portion 415b and lateral portions 415c. The implant 405 is made from a non-metal synthetic material with a metal plate 420 integrally formed within the anterior side of the non metal synthetic material implant 405. The metal plate 420 does not fully extend around the implant 405. The non-metal synthetic material may be made from carbon, PEEK (polyethylketone), graphite, woven carbon, Kevlar, or other suitable synthetic material that has strength capable of withstanding compression and rotational forces. The metal material may be titanium, stainless steel or cobaltlchromium. The amalgam feature could also be applied to NMSM threaded cages placed in the anterior lumbar spine, as well as cages placed in the interbody space from a lateral approach. This amalgam feature may also apply to cages, cylindrical or rectangular placed in the cervical or thoracic spine. While the preferred shape of the implant is oval, other shapes may also be used, such as circular, kidney or "D" shaped.

There are numerous advantages of a NMSM/metal amalgam for an implant. For example, one advantage is the metal within the device allows a surgeon to identify the position of the device in space to assist in implantation at the proper location and orientation with in the spine. Another advantage is that the NMSM material allows a surgeon to assess fusion postoperatively after the implantation of the device. This is due to the fact that x-rays penetrate the NMSM to allow bony visualization through the device. The surgeon would be able to evaluate the fusion of the device to the spine by using the lateral x-rays taken only through lateral portions of the NMSM device alone.

One weakness of using a NMSM device alone (i.e., without metal) is the difficulty in holding the device with instruments or less durable antirotation feature. Often the holding instruments (typically made of PEEK) overwhelm the NMSM device during implantation, resulting in deformation and damage. Another advantage of the disclosed NMSM/metal amalgam is that the metal can allow a firmer "grabbing" of the device with implantation tools. Holding or grabbing the proposed NMSM/metal amalgam device with an implantation tool, which would hold the metal plate(s), would avoid such damage to the implant and allow better control during implantation. Advantage of more durable feature to prevent rotation between the implant and plate. The combination of metal in the form of a fixation element within the NMSM device is a novel concept. In some embodiments, a plurality of tool engaging openings (not shown) may be disposed in the annular wall 415 having the metal plate(s) 420. The openings can be threaded or otherwise configured to receive a conventional insertion tool (not shown).

FIGs. 6B-6E show other embodiments of a NMSM/metal amalgam implant. In FIG. 6B, the metal within the device may include one or more metal plates, for example, plates 420a, 420b. In FIG. 6C, the metal within the NMSM/metal amalgam device could take the form of multiple washers or threaded inserts 425. In FIG. 6D, the metal within the NMSM/metal amalgam device could take the form of a plate 430 with threaded screw holes 435. In FIG. 6E, the metal within the NMSM/metal amalgam device could include both anterior plate(s) 420 on the anterior side and posterior plate(s) 440 on the posterior side of the device.

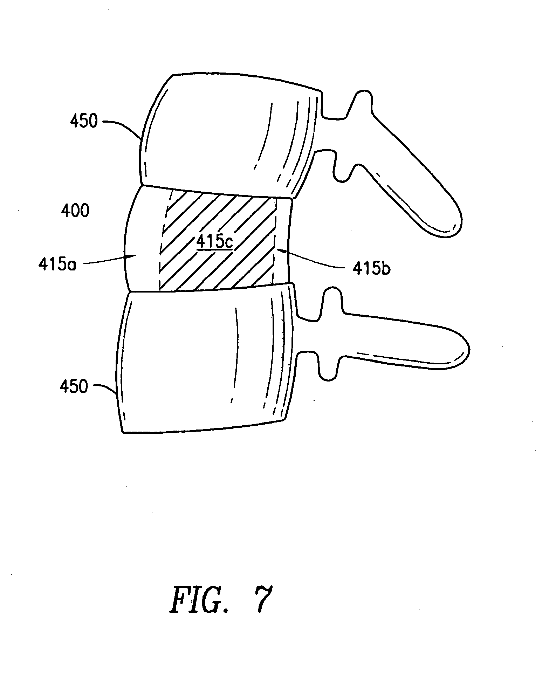

FIG. 7 shows a view of a lateral x-ray showing the implant 400 positioned within between adjacent vertebrae 450. By positioning the metal within the anterior portion 415a, and optionally the posterior portion 415b, the surgeon would be able to evaluate the fusion of the device to the spine by using the lateral x-rays taken only through lateral portions 415c of the NMSM device alone. An anterior 415a/posterior 415b x-ray would not be as desirable as the metal components of the device would obscure the fusion. There are numerous advantages of a NMSM/metal amalgam for an implant. For example, one advantage is the metal within the device allows a surgeon to identify the position of the device in space to assist in implantation at the proper location and orientation with in the spine. Another advantage is that the NMSM material allows a surgeon to assess fusion postoperatively after the implantation of the device. This is due to the fact that x-rays penetrate the NMSM to allow bony visualization through the device. Surgeons typically do not assess fusion through an anterior/posterior x-ray, and this is the view of the fusion that the metal components of the device would obscure.

Example embodiments of the methods and components of the present invention have been described herein. As noted elsewhere, these example embodiments have been described for illustrative purposes only, and are not limiting. Other embodiments are possible and are covered by the invention. Such embodiments will be apparent to persons skilled in the relevant art(s) based on the teachings contained herein. Thus, the breadth and scope of the present invention should not be limited by any of the above-described exemplary embodiments, but should be defined only in accordance with the following claims.

## Claims

1. A modular spinal implant system (300) for use between adjacent vertebrae near vascular anatomy, comprising
an implant (305) configured to fit between adjacent vertebrae, the implant having annular side walls (415) with upper and lower surfaces configured to enclose a hollow interior (410)
and
an attachment plate (310) rotatably coupled (315) to the implant and configured to rotate to variable orientations relative to the implant to avoid the vascular anatomy (325), the attachment plate having a superior portion that is narrower than an inferior portion, the attachment plate having at least one vertebra attachment hole configured for attaching to at least one adjacent vertebrae using one or more bone screws (320), **characterized in that** the implant comprises one or more metal plates (420) integrally formed within an anterior portion (415a) of the annular side wall;
and the attachment plate is rotatably coupled to the one or more metal plates within.

2. The system of claim 1, wherein the attachment plate is selected from a variety of attachment plates configured to avoid the vascular anatomy proximate the vertebrae.

3. The system of claim 1, wherein the inferior portion includes one or more vertebral attachment holes.

4. The system of claim 1, wherein the superior portion includes one vertebra attachment hole and the inferior portion includes two vertebra attachment holes.

5. The system of claim 1, wherein the implant material is selected from the group consisting of titanium, stainless steel, cobalt∼chromium, carbon, PEEK (polyethylketone), graphite, woven carbon, Kevlar, and other suitable synthetic material.

6. The system of claim 1, wherein the implant is made of a non metal synthetic material.

7. The system of claim 1, wherein the implant further includes one or more metal plates (440) integrally formed within posterior portion (415b) of the annular side wall.

8. The system of claim 1, wherein the attachment plate is made from titanium, stainless steel, or cobalt-chromium.

9. The system of claim 1, wherein mating surfaces between the attachment plate and implant include an interlock configuration.

10. The system of claim 1, further comprising a bone autograft, allograft or a bone graft substitute positioned within the hollow interior of the implant.

## Patentansprüche

1. Modulares Wirbelsäulen-Implantatsystem (300) zur Verwendung zwischen benachbarten Rückenwirbeln nahe einer Gefäßanatomie, umfassend
ein Implantat (305), das so ausgestaltet ist, dass es zwischen benachbarte Rückenwirbel passt, wobei das Implantat ringförmige Seitenwände (415) aufweist mit oberen und unteren Oberflächen, die so ausgestaltet sind, dass sie einen Innenhohlraum (410) umschließen, und
eine Befestigungsplatte (310), die mit dem Implantat drehbar gekoppelt ist (315) und so ausgestaltet, dass sie sich relativ zu dem Implantat in verschiedene Richtungen drehen kann, um die Gefäßanatomie (325) zu meiden, wobei die Befestigungsplatte einen höheren Teil aufweist, der schmäler ist als ein tieferer Teil, die Befestigungsplatte mindestens ein Rückenwirbelbefestigungsloch aufweist, das ausgestaltet ist für eine Befestigung an mindestens einem benachbarten Rückenwirbel unter Verwendung von einer oder mehreren Knochenschraube(n) (320), **dadurch gekennzeichnet, dass** das Implantat eine oder mehrere Metallplatten (420) umfasst, die integral mit einem vorderen Teil (415a) der ringförmigen Seitenwand ausgebildet ist bzw. sind, und die Befestigungsplatte drehbar gekoppelt ist mit der einen oder den mehreren Metallplatten.

2. System nach Anspruch 1, wobei die Befestigungsplatte ausgewählt ist aus einer Vielzahl an Befestigungsplatten, die so ausgestaltet sind, dass ein Annähren der Gefäßanatomie an die Rückenwirbel vermieden wird.

3. System nach Anspruch 1, wobei das tiefere Teil ein oder mehrere Rückenwirbelbefestigungslöcher aufweist.

4. System nach Anspruch 1, wobei das höhere Teil ein Rückenwirbelbefestigungsloch aufweist und das tiefere Teil zwei Rückenwirbelbefestigungslöcher aufweist.

5. System nach Anspruch 1, wobei das Implantatmaterial ausgewählt ist aus der Gruppe bestehend aus Titan, Edelstahl, Cobalt-Chrom, Carbon, PEEK (Polyethylketon), Graphit, Carbongewebe, Kevlar und anderen geeigneten synthetischen Materialien.

6. System nach Anspruch 1, wobei das Implantat aus einem synthetischen Nichtmetallmaterial hergestellt ist.

7. System nach Anspruch 1, wobei das Implantat ferner eine oder mehrere Metallplatte(n) (440) einschließt, die integral mit einem hinteren Teil (415b) der ringförmigen Seitenwand ausgebildet ist bzw. sind.

8. System nach Anspruch 1, wobei die Befestigungsplatte aus Titan, Edelstahl oder Cobalt-Chrom hergestellt ist.

9. System nach Anspruch 1, wobei Passflächen zwischen der Befestigungsplatte und dem Implantat eine verzahnende Konfiguration einschließen.

10. System nach Anspruch 1, ferner umfassend ein Knochenautotransplantat, -allotransplantat oder ein Knochentransplantat, das geeignet innerhalb des Innenhohlraums des Implantats positioniert ist.

## Revendications

1. Système d'implant spinal modulaire (300) à utiliser entre des vertèbres voisines, près de l'anatomie vasculaire, comprenant
un implant (305) conçu pour être placé de manière ajustée entre des vertèbres voisines, l'implant présentant des parois latérales annulaires (415) avec des surfaces supérieure et inférieure conçues pour entourer un espace intérieur creux (410), et
une plaque de fixation (310) reliée pivotante (315) à l'implant et conçue pour pivoter suivant des orientations variables par rapport à l'implant afin d'éviter l'anatomie vasculaire (325), la plaque de fixation présentant une partie supérieure qui est plus étroite qu'une partie inférieure, la plaque de fixation présentant au moins un trou de fixation pour vertèbre conçu pour une fixation à au moins une vertèbre voisine à l'aide d'une ou plusieurs vis pour os (320),
**caractérisé en ce que** l'implant comprend une ou plusieurs plaques de métal (420) formées d'une seule pièce avec une partie antérieure (415a) de la paroi latérale annulaire, et la plaque de fixation est reliée pivotante à la plaque ou aux plaques de métal, à l'intérieur.

2. Système de la revendication 1, étant précisé que la plaque de fixation est sélectionnée parmi une variété de plaques de fixation conçues pour éviter l'anatomie vasculaire près des vertèbres.

3. Système de la revendication 1, étant précisé que la partie inférieure présente un ou plusieurs trous de fixation pour vertèbre.

4. Système de la revendication 1, étant précisé que la partie supérieure présente un trou de fixation pour vertèbre, et que la partie inférieure présente deux trous de fixation pour vertèbre.

5. Système de la revendication 1, étant précisé que la matière d'implant est sélectionnée dans le groupe constitué par le titane, l'acier inoxydable, le cobalt∼chrome, le carbone, le PEEK (polyéthylcétone), le graphite, le carbone tissé, le Kevlar et d'autres matières synthétiques appropriées.

6. Système de la revendication 1, étant précisé que l'implant est en matière synthétique non métallique.

7. Système de la revendication 1, étant précisé que l'implant comprend par ailleurs une ou plusieurs plaques métalliques (440) formées d'une seule pièce à l'intérieur de la partie postérieure (415b) de la paroi latérale annulaire.

8. Système de la revendication 1, étant précisé que la plaque de fixation est en titane, en acier inoxydable ou en cobalt-chrome.

9. Système de la revendication 1, étant précisé que les surfaces d'accouplement entre la plaque de fixation et l'implant présentent une configuration de verrouillage mutuel.

10. Système de la revendication 1, comprenant par ailleurs une autogreffe ou allogreffe osseuse ou un substitut de greffe osseuse placé dans l'espace intérieur creux de l'implant.
